(19) Europäisches Patentamt
European Patent Office
Office européen des brevets

(11) EP 2 722 396 A1

(12) **EUROPEAN PATENT APPLICATION**
published in accordance with Art. 153(4) EPC

(43) Date of publication:
**23.04.2014 Bulletin 2014/17**

(21) Application number: **12800757.2**

(22) Date of filing: **14.06.2012**

(51) Int Cl.:
***C12Q 1/37*** *(2006.01)* ***C12Q 1/26*** *(2006.01)*
***C12Q 1/28*** *(2006.01)*

(86) International application number:
**PCT/JP2012/065237**

(87) International publication number:
**WO 2012/173185 (20.12.2012 Gazette 2012/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.06.2011 JP 2011134701**

(71) Applicant: **Kyowa Medex Co., Ltd.**
**Tokyo 104-6004 (JP)**

(72) Inventor: **SOYA, Haruyo**
**Sunto-gun**
**Shizuoka 411-0932 (JP)**

(74) Representative: **Vossius & Partner**
**Siebertstrasse 4**
**81675 München (DE)**

# (54) METHOD FOR MEASURING GLYCOSYLATED HEMOGLOBIN, MEASUREMENT REAGENT, AND MEASUREMENT KIT

(57) Described is a method for accurately and highly sensitively measuring glycated hemoglobin in a hemoglobin-containing sample without being influenced by hemoglobin. It is related to a method for measuring glycated hemoglobin in a hemoglobin-containing sample, comprising: reacting hemoglobin-containing sample with a protease in the presence of a surfactant; and then reacting the obtained reaction product with fructosyl peptide oxidase, wherein the latter reaction or both of the former reaction and the latter reaction are performed in the presence of a halogen oxide, and measuring the generated hydrogen peroxide. The method for measuring glycated hemoglobin in a hemoglobin-containing sample provided by the present invention is useful in, for example, the measurement of glycated hemoglobin useful in the diagnosis of diabetes mellitus.

EP 2 722 396 A1

## Description

### Technical Field

The present invention relates to a method, a reagent, and a kit for measuring glycated hemoglobin.

### Background Art

Glycated hemoglobin is a glycation product of hemoglobin in which glucose is bound thereto. Hemoglobin takes a tetrameric structure consisting of α and β chains. The glycated product of N terminus of β chain of hemoglobin is called hemoglobin A1c (hereinafter also referred to as HbAlc), which increases with increase in blood glucose level and as such, is measured as a diabetes mellitus marker in clinical laboratory examinations.

Known methods for measuring glycated hemoglobin include, for example, chromatography such as HPLC, electrophoresis, immunoassay using an antibody such as latex immunoagglutination assay, and enzymatic assay using an enzyme reactive to a glycated protein and an enzyme reactive to a glycated peptide and/or a glycated amino acid.

A known method for enzymatically measuring glycated hemoglobin (absorptiometry) comprises: first denaturing hemoglobin in a hemoglobin-containing sample using a denaturant; reacting the denatured hemoglobin with a protease; subsequently reacting the generated glycated peptide with glycated peptide oxidase; reacting the generated hydrogen peroxide with a chromogen capable of developing color by oxidation in the presence of a peroxidatively active substance such as peroxidase to convert the chromogen to a dye; and measuring the glycated hemoglobin on the basis of the absorbance of the generated dye.

This measurement of glycated hemoglobin based on absorptiometry is disadvantageously susceptible to influence of hemoglobin present in large amounts in the sample. For example, a method using a cationic surfactant and/or an amphoteric surfactant (Patent Document 1), a method using a sulfone compound and/or a nitro compound (Patent Documents 2 and 3), a method using a tetrazolium compound (Patent Document 4), and a method using a particular anionic surfactant such as polyoxyethylene alkyl ether sulfates (Patent Document 5) are known as solutions to this problem.

Unfortunately, these methods cannot always avoid the influence of hemoglobin. There is a demand for a method for accurately measuring glycated hemoglobin in a hemoglobin-containing sample without being influenced by hemoglobin.

Meanwhile, a method for measuring a substrate and an enzyme on the basis of redox reaction is known to employ iodate in order to avoid interference with a reducing agent, particularly, ascorbic acid (Patent Document 6). Nonetheless, use of a halogen oxide such as iodate for avoiding the influence of hemoglobin has not been known so far.

### Prior Art Documents

### Patent Documents

Patent Document 1: Japanese unexamined Patent Application Publication No. 3-010696
Patent Document 2: WO2003/107011
Patent Document 3: Japanese unexamined Patent Application Publication No. 2007-147630
Patent Document 4: Japanese unexamined Patent Application Publication No. 2000-210100
Patent Document 5: WO2005/049858
Patent Document 6: Japanese unexamined Patent Application Publication No. 56-151358


### Summary of the Invention

### Problems to be Solved by the Invention

An object of the present invention is to provide a method and a reagent for accurately and highly sensitively measuring glycated hemoglobin in a hemoglobin-containing sample without being influenced by hemoglobin.

### Means to Solve the Object

As a result of conducting diligent studies, the present inventor has found that a method comprises reacting a hemoglobin-containing sample with a protease in the presence of a surfactant and then reacting the obtained reaction

product with fructosyl peptide oxidase, wherein the latter reaction or both of the former reaction and the latter reaction are performed in the presence of a halogen oxide; and measuring the generated hydrogen peroxide, whereby glycated hemoglobin in the hemoglobin-containing sample can be measured accurately and highly sensitively without being influenced by hemoglobin. On the basis of these findings, the present invention has been completed. Specifically, the present invention relates to the following [1] to [18]:

[1] A method for measuring glycated hemoglobin in a hemoglobin-containing sample comprising: reacting the hemoglobin-containing sample with a protease in the presence of a surfactant, then reacting the obtained reaction product with fructosyl peptide oxidase, wherein the latter reaction or both of the former reaction and the latter reaction are performed in the presence of a halogen oxide, and measuring the generated hydrogen peroxide.
[2] The method according to [1], wherein the halogen oxide is a halogen oxide selected from the group consisting of iodic acid or a salt thereof, bromic acid or a salt thereof, and periodic acid or a salt thereof.
[3] The method according to [1] or [2], wherein the surfactant is a cationic surfactant.
[4] The method according to [3], wherein the cationic surfactant is a cationic surfactant selected from the group consisting of a pyridinium salt represented by the following formula (I), a phosphonium salt represented by the following formula (II), and a quaternary ammonium salt represented by the following formula (III):

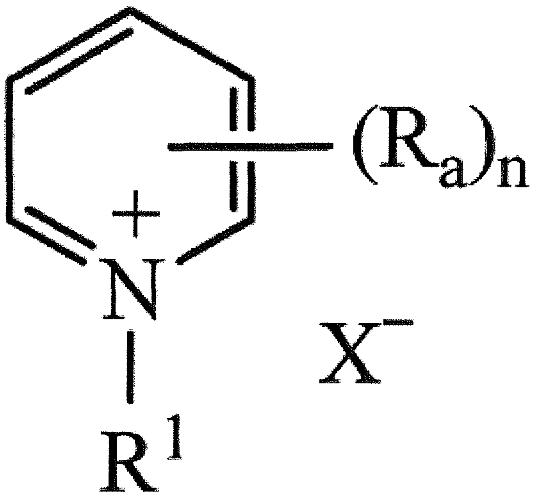

(I)

wherein $R^1$ represents a substituted or unsubstituted alkyl or a substituted or unsubstituted alkenyl; $R_a$ represents a hydrogen atom, a substituted or unsubstituted alkyl, or a substituted or unsubstituted alkenyl; n represents an integer of 1 to 5; and $X^-$ represents a monovalent anion,

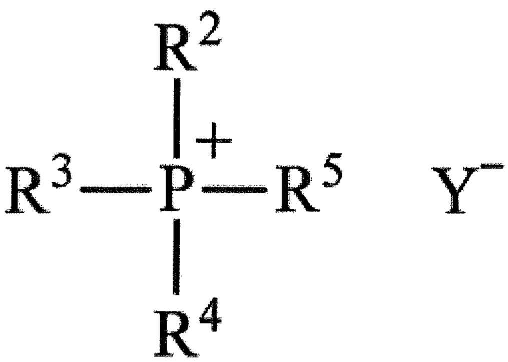

(II)

wherein $R^2$ to $R^5$ are the same or different, and each represents a substituted or unsubstituted alkyl; and $Y^-$ represents a monovalent anion, and

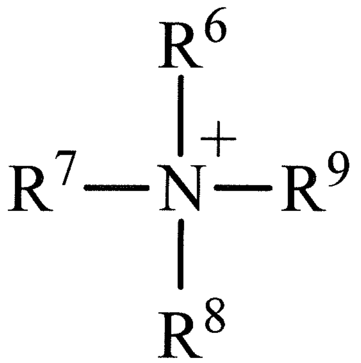 $Z^-$ ( III )

wherein $R^6$ to $R^9$ are the same or different, and each represents a substituted or unsubstituted alkyl; and Zrepresents a monovalent anion.

[5] The method according to any one of [1] to [4], wherein the measurement of the hydrogen peroxide is performed using a reagent for measuring hydrogen peroxide.

[6] The method according to [5], wherein the reagent for measuring hydrogen peroxide is a reagent comprising peroxidase and a leuco chromogen.

[7] A reagent for measuring glycated hemoglobin in a hemoglobin-containing sample comprising a protease, fructosyl peptide oxidase, a halogen oxide, and a surfactant.

[8] The reagent according to [7], wherein the halogen oxide is a halogen oxide selected from the group consisting of iodic acid or a salt thereof, bromic acid or a salt thereof, and periodic acid or a salt thereof.

[9] The reagent according to [7] or [8], wherein the surfactant is a cationic surfactant.

[10] The reagent according to [9], wherein the cationic surfactant is a cationic surfactant selected from the group consisting of a pyridinium salt represented by the following formula (I), a phosphonium salt represented by the following formula (II), and a quaternary ammonium salt represented by the following formula (III):

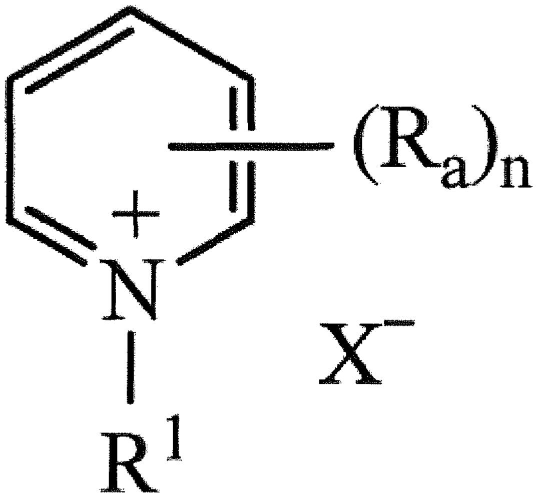 ( I )

wherein $R^1$ represents a substituted or unsubstituted alkyl or a substituted or unsubstituted alkenyl; $R_a$ represents a hydrogen atom, a substituted or unsubstituted alkyl, or a substituted or unsubstituted alkenyl; n represents an integer of 1 to 5; and $X^-$ represents a monovalent anion,

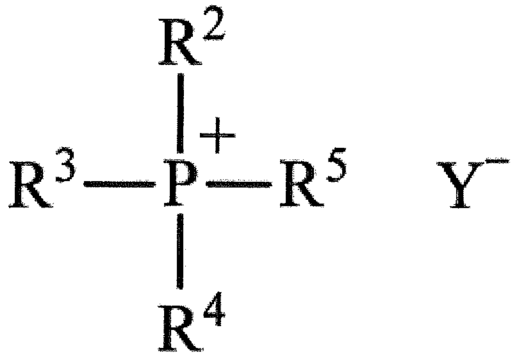

(II)

wherein $R^2$ to $R^5$ are the same or different, and each represents a substituted or unsubstituted alkyl; and Yrepresents a monovalent anion, and

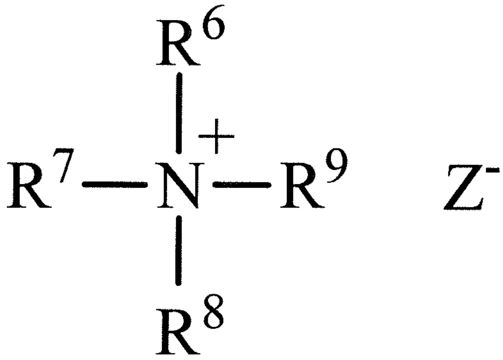

(III)

wherein $R^6$ to $R^9$ are the same or different, and each represents a substituted or unsubstituted alkyl; and Zrepresents a monovalent anion.

[11] The reagent according to any one of [7] to [10], further comprising a reagent for measuring hydrogen peroxide.

[12] The reagent according to [11], wherein the reagent for measuring hydrogen peroxide is a reagent comprising peroxidase and a leuco chromogen.

[13] A kit for measuring glycated hemoglobin in a hemoglobin-containing sample comprising: a first reagent comprising a protease, a halogen oxide, and a surfactant; and a second reagent comprising fructosyl peptide oxidase.

[14] A kit for measuring glycated hemoglobin in a hemoglobin-containing sample comprising: a first reagent comprising a protease and a surfactant; and a second reagent comprising fructosyl peptide oxidase and a halogen oxide.

[15] The kit according to [13] or [14], wherein the halogen oxide is a halogen oxide selected from the group consisting of iodic acid or a salt thereof, bromic acid or a salt thereof, and periodic acid or a salt thereof.

[16] The kit according to any one of [13] to [15], wherein the surfactant is a cationic surfactant.

[17] The kit according to [16], wherein the cationic surfactant is a cationic surfactant selected from the group consisting of a pyridinium salt represented by the following formula (I), a phosphonium salt represented by the following formula (II), and a quaternary ammonium salt represented by the following formula (III):

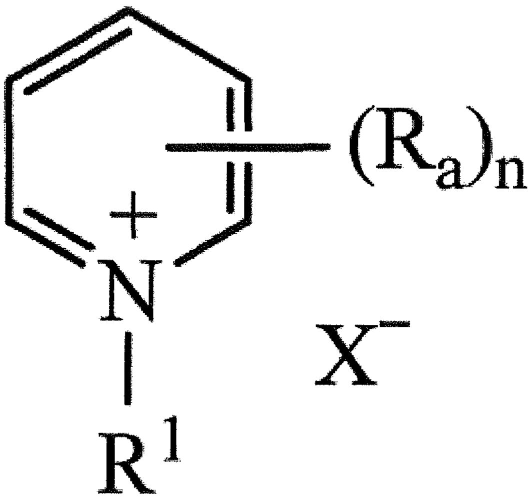

( I )

wherein $R^1$ represents a substituted or unsubstituted alkyl or a substituted or unsubstituted alkenyl; $R_a$ represents a hydrogen atom, a substituted or unsubstituted alkyl, or a substituted or unsubstituted alkenyl; n represents an integer of 1 to 5; and $X^-$ represents a monovalent anion,

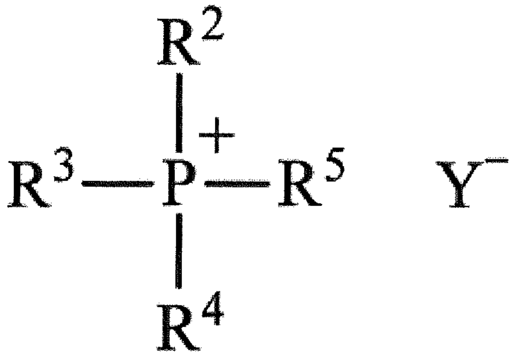

( II )

wherein $R^2$ to $R^5$ are the same or different, and each represents a substituted or unsubstituted alkyl; and Yrepresents a monovalent anion,

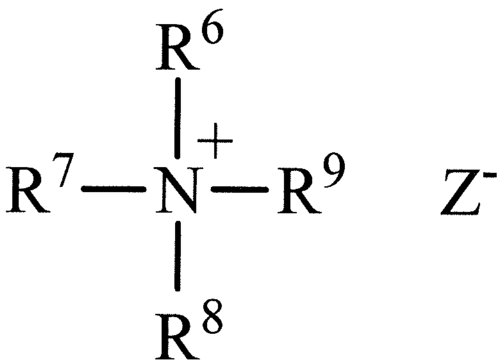

( III )

wherein $R^6$ to $R^9$ are the same or different, and each represents a substituted or unsubstituted alkyl; and Zrepresents a monovalent anion, and

[18] The kit according to any one of [13] to [17], wherein each of peroxidase and a leuco chromogen is comprised in the first reagent and the second reagent, or the second reagent and the first reagent, respectively.

## Effect of the Invention

The present invention provides a method, a reagent, and a kit for accurately and highly sensitively measuring

glycated hemoglobin in a hemoglobin-containing sample without being influenced by hemoglobin.

## Brief Description of Drawings

[Figure 1] Figure 1 is a graph showing the relationship between hemoglobin concentration and reaction absorbance in the assay of HbA1c in a specimen using kits of Examples 1 to 3 and Comparative Example 1. The symbol ♦ represents the results of measurement using the kit of Comparative Example 1. The symbol ○ represents the results of measurement using the kit of Example 1. The symbol A represents the results of measurement using the kit of Example 2. The symbol D represents the results of measurement using the kit of Example 3. The ordinate represents reaction absorbance ($\times 10^{-4}$ Abs). The abscissa represents hemoglobin concentration (mg/mL).

[Figure 2] Figure 2 is a graph showing the relationship between hemoglobin concentration and reaction absorbance in the measurement of HbA1c in a specimen using kits of Example 4 and Comparative Example 2. The symbol ♦ represents the results of measurement using the kit of Comparative Example 2. The symbol D represents the results of measurement using the kit of Example 4. The ordinate represents reaction absorbance ($\times 10^{-4}$ Abs). The abscissa represents hemoglobin concentration (mg/mL).

[Figure 3] Figure 3 is a graph showing the relationship between hemoglobin concentration and reaction absorbance in the measurement of HbA1c in a specimen using kits of Example 5 and Comparative Example 3. The symbol ♦ represents the results of measurement using the kit of Comparative Example 3. The symbol □ represents the results of measurement using the kit of Example 5. The ordinate represents reaction absorbance ($\times 10^{-4}$ Abs). The abscissa represents hemoglobin concentration (mg/mL).

[Figure 4] Figure 4 is a graph showing the relationship between hemoglobin concentration and reaction absorbance in the measurement of HbA1c in a specimen using kits of Example 6 and Comparative Example 4. The symbol ♦ represents the results of measurement using the kit of Comparative Example 4. The symbol ○ represents the results of measurement using the kit of Example 6. The ordinate represents reaction absorbance ($\times 10^{-4}$ Abs). The abscissa represents hemoglobin concentration (mg/mL).

## Mode for Carrying Out the Invention

(1) Method for measuring glycated hemoglobin in hemoglobin-containing sample

The method for measuring glycated hemoglobin in a hemoglobin-containing sample according to the present invention comprises: reacting glycated hemoglobin in the hemoglobin-containing sample with a protease in the presence of a surfactant; and then reacting the obtained reaction product with fructosyl peptide oxidase, wherein the latter reaction or both of the former reaction and the latter reaction are performed in the presence of a halogen oxide, and measuring the generated hydrogen peroxide. The halogen oxide can be allowed to exist in the reaction of fructosyl peptide oxidase or can be allowed to exist in both of the reaction of a protease and the reaction of fructosyl peptide oxidase.

Specific examples of the measuring method include measuring methods comprising the following steps:

<Measuring method 1>

(1) a step of reacting glycated hemoglobin with a protease in the hemoglobin-containing sample in the presence of a surfactant;

(2) a step of reacting the reaction product obtained in step (1) with fructoxyl peptide in the presence of a halogen oxide to generate hydrogen peroxide;

(3) a step of measuring the hydrogen peroxide generated in step (2); and

(4) a step of determining the concentration of glycated hemoglobin in the hemoglobin-containing sample from the amount of the hydrogen peroxide measured in step (3) on the basis of a calibration curve representing the relationship between the amount of hydrogen peroxide and the concentration of glycated hemoglobin, prepared in advance using known concentrations of glycated hemoglobin.

<Measuring method 2>

(1) a step of reacting glycated hemoglobin in the hemoglobin-containing sample with a protease in the presence of a halogen oxide and a surfactant;
(2) a step of reacting the reaction product obtained in step (1) with fructosyl peptide oxidase to generate hydrogen peroxide;
(3) a step of measuring the hydrogen peroxide formed in step (2); and
(4) a step of determining the concentration of glycated hemoglobin in the hemoglobin-containing sample from the amount of the hydrogen peroxide measured in step (3) on the basis of a calibration curve representing the relationship between the amount of hydrogen peroxide and the concentration of glycated hemoglobin, prepared in advance using known concentrations of glycated hemoglobin.

The method for measuring glycated hemoglobin in a hemoglobin-containing sample according to the present invention also encompasses even a method involving calculating the ratio of the amount of glycated hemoglobin to the amount of total hemoglobin (i.e., total hemoglobin composed of hemoglobin and glycated hemoglobin) in the hemoglobin-containing sample. In this case, a method for measuring glycated hemoglobin in a hemoglobin-containing sample according to the present invention specifically comprises the following steps:

<Measuring method 3>

(1) a step of determining the amount of total hemoglobin (i.e., total hemoglobin composed of hemoglobin and glycated hemoglobin) in the hemoglobin-containing sample;
(2) a step of reacting glycated hemoglobin in the hemoglobin-containing sample with a protease in the presence of a surfactant;
(3) a step of reacting the reaction product obtained in step (2) with fructosyl peptide oxidase in the presence of a halogen oxide to generate hydrogen peroxide;
(4) a step of measuring the hydrogen peroxide generated in step (3); and
(5) a step of determining the amount of glycated hemoglobin in the hemoglobin-containing sample from the amount of the hydrogen peroxide measured in step (4) on the basis of a calibration curve representing the relationship between the amount of hydrogen peroxide and the amount of glycated hemoglobin, prepared in advance using known amounts of glycated hemoglobin; and
(6) a step of calculating the ratio of the amount of glycated hemoglobin to the amount of total hemoglobin in the hemoglobin-containing sample from the amount of total hemoglobin determined in step (1) and the amount of glycated hemoglobin determined in step (5).

Step (1) of determining the amount of total hemoglobin can be performed after step (2).

<Measuring method 4>

(1) a step of determining the amount of total hemoglobin (i.e., total hemoglobin composed of hemoglobin and glycated hemoglobin) in the hemoglobin-containing sample;
(2) a step of reacting glycated hemoglobin in the hemoglobin-containing sample with a protease in the presence of a halogen oxide and a surfactant;
(3) a step of reacting the reaction product obtained in step (2) with fructosyl peptide oxidase to generate hydrogen peroxide;
(4) a step of measuring the hydrogen peroxide generated in step (3);
(5) a step of determining the amount of glycated hemoglobin in the hemoglobin-containing sample from the amount of the hydrogen peroxide measured in step (4) on the basis of a calibration curve representing the relationship between the amount of hydrogen peroxide and the amount of glycated hemoglobin, prepared in advance using known amounts of glycated hemoglobin; and
(6) a step of calculating the ratio of the amount of glycated hemoglobin to the amount of total hemoglobin in the hemoglobin-containing sample from the amount of total hemoglobin determined in step (1) and the amount of glycated hemoglobin determined in step (5).

Step (1) of determining the amount of total hemoglobin can be performed after step (2).

The hemoglobin-containing sample used in the measuring method of the present invention is not particularly limited as long as the sample contains hemoglobin and is applicable to the method for measuring glycated hemoglobin according to the present invention. Examples thereof include whole blood, blood cells, mixed samples of blood cells and plasma, and hemolyzed samples of these samples. The hemolyzing treatment is not particularly limited as long as the treatment hemolyzes whole blood, blood cells, or mixed samples of blood cells and plasma. Examples thereof include physical, chemical, and biological methods. Examples of the physical method include a method using a hypotonic solution such as distilled water, and a method using sonic waves. Examples of the chemical method include a method using an organic solvent such as methanol, ethanol, or acetone, and a method using a polyoxyethylene surfactant. Examples of the biological method include a method using an antibody or a complement.

The glycated hemoglobin according to the present invention is generated by binding of a sugar such as glucose to hemoglobin. Examples thereof include hemoglobin A1a, hemoglobin A1b, and hemoglobin A1c. Hemoglobin A1c is preferable.

The halogen oxide according to the present invention is not particularly limited as long as the halogen oxide enables the method for measuring glycated hemoglobin according to the present invention. Examples thereof include iodic acid or a salt thereof, bromic acid or a salt thereof, and periodic acid or a salt thereof. Examples of the salt include lithium salt, sodium salt, potassium salt, ammonium salt, calcium salt, and magnesium salt.

Specific examples (products) of the halogen oxide include iodic acid, sodium iodate, potassium iodate, bromic acid, sodium bromate, potassium bromate, periodic acid, sodium periodate, and potassium periodate.

In the method for measuring glycated hemoglobin according to the present invention, the concentration of the halogen oxide in the reaction solution is not particularly limited as long as the concentration enables the method for measuring glycated hemoglobin according to the present invention. The concentration is usually 0.005 to 20 mmol/L, preferably 0.01 to 10 mmol/L.

The surfactant according to the present invention is not particularly limited as long as the surfactant enables the method for measuring glycated hemoglobin according to the present invention. Examples thereof include a cationic surfactant, an anionic surfactant, an amphoteric surfactant, and a nonionic surfactant. A cationic surfactant is preferable.

Examples of the cationic surfactant include a pyridinium salt, a phosphonium salt, an imidazolium salt, a quaternary ammonium salt, and an isoquinolinium salt. A pyridinium salt, a phosphonium salt, or a quaternary ammonium salt is preferable.

A pyridinium salt represented by the following formula (I) [hereinafter, referred to as compound (I)] is used as the pyridinium salt:

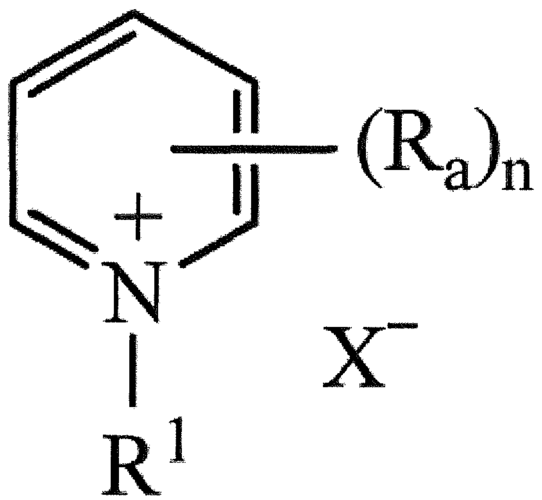

(I)

In the formula, $R^1$ represents a substituted or unsubstituted alkyl or a substituted or unsubstituted alkenyl; $R_a$ represents a hydrogen atom, a substituted or unsubstituted alkyl, or a substituted or unsubstituted alkenyl; n represents an integer of 1 to 5; and Xrepresents a monovalent anion, respectively.

Examples of alkyl in the substituted or unsubstituted alkyl represented by $R^1$ include linear alkyl having 1 to 20 carbon atoms, and branched alkyl having 3 to 20 carbon atoms. Examples of the linear alkyl having 1 to 20 carbon atoms include methyl, ethyl, propyl, butyl, pentyl, hexyl, heptyl, octyl, nonyl, decyl, undecyl, dodecyl (lauryl), tridecyl, tetradecyl (myristyl), pentadecyl, hexadecyl (cetyl), heptadecyl, octadecyl (stearyl), nonadecyl, and icosyl. Examples of the branched alkyl having 3 to 20 carbon atoms include isopropyl, isobutyl, isopentyl, isohexyl, isoheptyl, isooctyl, isononyl,

isodecyl, isoundecyl, isododecyl, isotridecyl, isotetradecyl, isopentadecyl, isohexadecyl, isoheptadecyl, isooctadecyl, isononadecyl, isoicosyl, and octyldodecyl.

Examples of alkenyl in the substituted or unsubstituted alkenyl represented by $R^1$ include alkenyl having 2 to 20 carbon atoms. Examples of the alkenyl having 2 to 20 carbon atoms include vinyl, propenyl, allyl, butenyl, pentenyl, hexenyl, heptenyl, octenyl, nonenyl, decenyl, undecenyl, dodecenyl, tetradecenyl, pentadecenyl, hexadecenyl, heptadecenyl, octadecenyl, oleyl, nonadecenyl, and icosenyl.

Examples of the substituent in the substituted alkyl or the substituted alkenyl represented by $R^1$ include a phenyl group, a hydroxy group, a sulfo group, a cyano group, and a halogen atom. Examples of the halogen atom include chlorine atom, bromine atom, and iodine atom.

Examples of alkyl in the substituted or unsubstituted alkyl represented by $R_a$ include linear alkyl having 1 to 20 carbon atoms, and branched alkyl having 3 to 20 carbon atoms. Examples of the linear alkyl having 1 to 20 carbon atoms include those exemplified above as the linear alkyl having 1 to 20 carbon atoms. Examples of the branched alkyl having 3 to 20 carbon atoms include those exemplified above as the branched alkyl having 3 to 20 carbon atoms.

Examples of alkenyl in the substituted or unsubstituted alkenyl represented by $R_a$ include alkenyl having 2 to 20 carbon atoms. Examples of the alkenyl having 2 to 20 carbon atoms include those exemplified above as the linear alkenyl having 2 to 20 carbon atoms.

Examples of the substituent in the substituted alkyl or the substituted alkenyl represented by $R_a$ include a phenyl group, a hydroxy group, a sulfo group, a cyano group, and halogen atoms. Examples of the phenyl group-substituted alkyl include benzyl and 1-phenylethyl. Examples of the halogen atom include chlorine atom, bromine atom, and iodine atom.

When the pyridine ring has two or more substituents, these substituents may be the same or different. $X^-$ in compound (I) represents a monovalent anion. Examples of the monovalent anion include anions such as a halogen ion, $OH^-$, $PF_6^-$, $BF_4^-$, $CH_3CH_2OSO_3^-$, and $(CF_3SO_2)_2N^-$. Examples of the halogen ion include $Cl^-$, $Br^-$, and $I^-$.

Specific examples (products) of compound (I) include 1-dodecylpyridinium chloride (hereinafter, referred to as C12py; manufactured by Tokyo Chemical Industry Co., Ltd.), 1-cetylpyridinium chloride (hereinafter, referred to as C16py; manufactured by Tokyo Chemical Industry Co., Ltd.), 1-cetyl-4-methylpyridinium chloride (manufactured by Tokyo Chemical Industry Co., Ltd.), and N-octadecyl-4-stilbazole bromide (manufactured by Tokyo Chemical Industry Co., Ltd.).

A phosphonium salt represented by the following formula (II) [hereinafter, referred to as compound (II)] is used as the phosphonium salt;

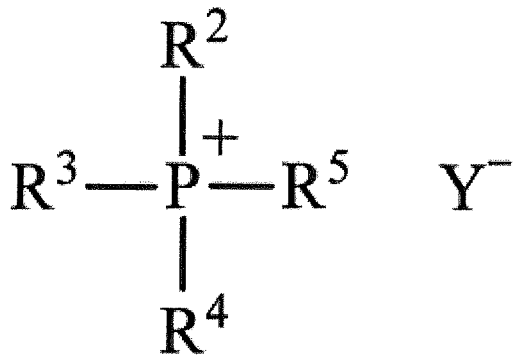

(II)

In the formula, $R^2$ to $R^5$ are the same or different, and each represents a substituted or unsubstituted alkyl; and $Y^-$ represents a monovalent anion, respectively.

Examples of alkyl in the substituted or unsubstituted alkyl represented by $R^2$ include linear alkyl having 8 to 20 carbon atoms, and branched alkyl having 8 to 20 carbon atoms. Examples of the linear alkyl having 8 to 20 carbon atoms include octyl, nonyl, decyl, undecyl, dodecyl (lauryl), tridecyl, tetradecyl (myristyl), pentadecyl, hexadecyl (cetyl), heptadecyl, octadecyl (stearyl), nonadecyl, and icosyl. Examples of the branched alkyl having 8 to 20 carbon atoms include isooctyl, isononyl, isodecyl, isoundecyl, isododecyl, isotridecyl, isotetradecyl, isopentadecyl, isohexadecyl, isoheptadecyl, isooctadecyl, isononadecyl, isoicosyl, and octyldodecyl. Examples of the substituent in the substituted alkyl include a phenyl group, a hydroxy group, a sulfo group, a cyano group, and a halogen atom. Examples of the phenyl group-substituted alkyl include benzyl and 1-phenylethyl. Examples of the halogen atom include chlorine atom, bromine atom, and iodine atom.

Examples of alkyl in the substituted or unsubstituted alkyl represented by each of $R^3$ to $R^5$ include linear alkyl having 1 to 20 carbon atoms, and branched alkyl having 3 to 20 carbon atoms. Examples of the linear alkyl having 1 to 20 carbon atoms include those exemplified above as the linear alkyl having 1 to 20 carbon atoms. Examples of the branched alkyl having 3 to 20 carbon atoms include those exemplified above as the branched alkyl having 3 to 20 carbon

atoms. Examples of the substituent in the substituted alkyl include a phenyl group, a hydroxy group, a sulfo group, a cyano group, and a halogen atom. Examples of the phenyl group-substituted alkyl include benzyl and 1-phenylethyl. Examples of the halogen atom include chlorine atom, bromine atom, and iodine atom.

$Y^-$ represents a monovalent anion. Examples of the monovalent anion include anions such as a halogen ion, $OH^-$, $PF_6^-$, $BF_4^-$, $CH_3CH_2OSO_3^-$, $(CF_3SO_2)_2N^-$, $B(C_6H_5)_4$, and benzotriazolate. Examples of the halogen ion include $Cl^-$, $Br^-$, and $I^-$.

Specific examples (products) of compound (II) include tributyldodecylphosphonium bromide (hereinafter, referred to as C12TBP; manufactured by Tokyo Chemical Industry Co., Ltd.), tributylhexadecylphosphonium bromide (manufactured by Tokyo Chemical Industry Co., Ltd.), tetraoctylphosphonium bromide (manufactured by Tokyo Chemical Industry Co., Ltd.), and tributyloctylphosphonium bromide (manufactured by Tokyo Chemical Industry Co., Ltd.).

A quaternary ammonium salt represented by the following formula (III) [hereinafter, referred to as compound (III)] is used as the quaternary ammonium salt:

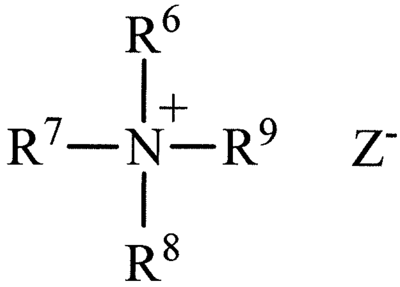

(III)

In the formula, $R^6$ to $R^9$ are the same or different, and each represents a substituted or unsubstituted alkyl; and $Z^-$ represents a monovalent anion.

Examples of alkyl in the substituted or unsubstituted alkyl represented by $R^6$ include linear alkyl having 8 to 20 carbon atoms, and branched alkyl having 8 to 20 carbon atoms. Examples of the linear alkyl having 8 to 20 carbon atoms include those exemplified above as the linear alkyl having 8 to 20 carbon atoms. Examples of the branched alkyl having 8 to 20 carbon atoms include those exemplified above as the branched alkyl having 8 to 20 carbon atoms. Examples of the substituent in the substituted alkyl include a phenyl group, a hydroxy group, a sulfo group, a cyano group, and a halogen atom. Examples of the phenyl group-substituted alkyl include benzyl and 1-phenylethyl. Examples of the halogen atom include chlorine atom, bromine atom, and iodine atom.

Examples of alkyl in the substituted or unsubstituted alkyl represented by each of $R^7$ to $R^9$ include linear alkyl having 1 to 20 carbon atoms, and branched alkyl having 3 to 20 carbon atoms. Examples of the linear alkyl having 1 to 20 carbon atoms include those exemplified above as the linear alkyl having 1 to 20 carbon atoms. Examples of the branched alkyl having 3 to 20 carbon atoms include those exemplified above as the branched alkyl having 3 to 20 carbon atoms. Examples of the substituent in the substituted alkyl include a phenyl group, a hydroxy group, a sulfo group, a cyano group, and a halogen atom. Examples of the phenyl group-substituted alkyl include benzyl and 1-phenylethyl. Examples of the halogen atom include chlorine atom, bromine atom, and iodine atom.

$Z^-$ represents a monovalent anion. Examples of the monovalent anion include anions such as a halogen ion, $OH^-$, $PF_6^-$, $BF_4^-$, $CH_3CH_2OSO_3^-$, $(CF_3SO_2)_2N^-$, $B(C_6H_5)_4^-$ and benzotriazolate. Examples of the halogen ion include $Cl^-$, $Br^-$, and $I^-$.

Specific examples (products) of compound (III) include decyltrimethylammonium chloride, decyltrimethylammonium bromide, dodecyltrimethylammonium chloride, dodecyltrimethylammonium bromide, tetradecyltrimethylammonium chloride (hereinafter, referred to as C14TMA), tetradecyltrimethylammonium bromide, hexadecyltrimethylammonium chloride, hexadecyltrimethylammonium bromide, didecyldimethylammonium chloride, didecyldimethylammonium bromide, didodecyldimethylammonium chloride, and didodecyldimethylammonium bromide (all manufactured by Tokyo Chemical Industry Co., Ltd.).

Examples of the anionic surfactant include a sulfuric acid ester salt, a carboxylate, sulfonate, a phosphoric acid ester salt, a sulfosuccinate, an N-methyltaurine salt, and an N-alkanoyl-N-methyltaurine salt.

Examples of the amphoteric surfactants include a tertiary amine oxide and an alkylcarboxybetaine.

Examples of the nonionic surfactant include polyoxyethylene alkylamine, polyoxyethylene alkenylamine, polyoxyethylene alkyl ether, polyoxyethylene alkenyl ether, polyoxyethylene alkylphenyl ether, ethylenediamine tetrapolyoxyethylene, and polyglycerin fatty acid ester.

The amount of total hemoglobin can be determined by a method known in the art, for example, a cyanmethemoglobin method, an oxyhemoglobin method, or an SLS-hemoglobin method. The amount of total hemoglobin can be determined by applying the cyanmethemoglobin method, the oxyhemoglobin method, or the SLS-hemoglobin method

not only to the hemoglobin-containing sample itself but to a hemoglobin-containing sample added with a halogen oxide and/or a surfactant or a hemoglobin-containing sample added with a halogen oxide and/or a surfactant and a protease.

The reaction of glycated hemoglobin in the hemoglobin-containing sample with a protease in the presence of the surfactant can be performed under any condition as long as the protease can react to glycated hemoglobin in the presence of the surfactant. The reaction of glycated hemoglobin in the hemoglobin-containing sample with a protease is preferably performed in an aqueous medium. Examples of the aqueous medium include an aqueous medium described later. The reaction of glycated hemoglobin in the hemoglobin-containing sample with a protease is performed usually at 10 to 50°C, preferably 20 to 40°C, and usually for 1 minute to 3 hours, preferably 2.5 minutes to 1 hour. The concentration of the protease is not particularly limited as long as the reaction of glycated hemoglobin in the hemoglobin-containing sample with the protease proceeds. The concentration is usually 50 to 25,000 kU/L, preferably 250 to 10,000 kU/L.

The protease is not particularly limited as long as the protease reacts to glycated hemoglobin in the hemoglobin-containing sample to generate a glycated peptide from the glycated hemoglobin. Examples thereof include a serine protease (chymotrypsin, subtilisin, etc.), a cysteine protease (papain, caspase, etc.), an aspartic acid protease (pepsin, cathepsin D, etc.), a metalloprotease (thermolysin, etc.), an N-terminal threonine protease, and a glutamic acid protease. In the present invention, a commercially available protease can be used. Examples of the commercially available product include Protease P "Amano" 3G and Protease K "Amano" (both manufactured by Amano Enzyme Inc.), Actinase AS and Actinase E (both manufactured by Kaken Pharma Co., Ltd.), Thermolysin (manufactured by Daiwa Fine Chemicals Co., Ltd.), and Sumizyme MP (manufactured by Shin Nihon Chemical Co., Ltd.).

The concentration of the surfactant in the reaction of a protease is not particularly limited as long as the reaction of glycated hemoglobin in the hemoglobin-containing sample with the protease proceeds. The concentration is usually 0.0001 to 10%, preferably 0.0005 to 5%.

The reaction of glycated hemoglobin in the hemoglobin-containing sample with the protease forms a reaction product comprising a glycated peptide. Subsequently, this glycated peptide in the reaction product reacts with fructosyl peptide oxidase to generate hydrogen peroxide. The reaction of the glycated peptide with fructosyl peptide oxidase is preferably performed in an aqueous medium. Examples of the aqueous medium include an aqueous medium described later.

The reaction of the glycated peptide with fructosyl peptide oxidase is performed usually at 10 to 50°C, preferably 20 to 40°C, and usually for 1 minute to 3 hours, preferably 2.5 minutes to 1 hour. The concentration of the fructosyl peptide oxidase is not particularly limited as long as the reaction of the glycated hemoglobin with the fructosyl peptide oxidase proceeds. The concentration is usually 0.1 to 30 kU/L, preferably 0.2 to 15 kU/L.

The fructosyl peptide oxidase is not particularly limited as long as the oxidase acts on the glycated peptide to generate hydrogen peroxide. Examples thereof include fructosyl peptide oxidases derived from filamentous bacteria, yeasts, actinomycetes, bacteria, or archaebacteria. In the present invention, commercially available fructosyl peptide oxidase can be used. Examples of the commercially available product include FPOX-CE (manufactured by Kikkoman Corp.), FPOX-EE (manufactured by Kikkoman Corp.), and FPOX-CET (manufactured by Kikkoman Corp.).

Examples of the method for measuring the generated hydrogen peroxide include a method using an electrode, and a method using a reagent for measuring hydrogen peroxide. A method using a reagent for measuring hydrogen peroxide is preferable. The reagent for measuring hydrogen peroxide refers to a reagent for converting hydrogen peroxide to a detectable substance. Examples of the detectable substance include a dye, a light (luminescence), and a fluorescence. A dye is preferable.

In the case the detectable substance is a dye, examples of the reagent for measuring hydrogen peroxide include a reagent comprising a peroxidatively active substance such as peroxidase and a chromogen capable of developing color by oxidation. Examples of the chromogen capable of developing color by oxidation include an oxidative coupling-type chromogen and a leuco chromogen. A leuco chromogen is preferable. Examples of the leuco chromogen include a phenothiazine chromogen, a triphenylmethane chromogen, a diphenylamine chromogen, o-phenylenediamine, hydroxypropionic acid, diaminobenzidine, and tetramethylbenzidine. A phenothiazine chromogen is preferable. Examples of the phenothiazine chromogen include 10-N-carboxymethylcarbamoyl-3,7-bis(dimethylamino)-10H-phenothiazine (CCAP), 10-N-methylcarbamoyl-3,7-bis(dimethylamino)-10H-phenothiazine (MCDP), and 10-N-(carboxymethylaminocarbonyl)-3,7-bis(dimethylamino)-10H-phenothiazine sodium salt (DA-67). Among these phenothiazine chromogens, 10-N-(carboxymethylaminocarbonyl)-3,7-bis(dimethylamino)-10H-phenothiazine sodium salt (DA-67) is particularly preferable. Examples of the triphenylmethane chromogen include N,N,N',N',N",N"-hexa(3-sulfopropyl)-4,4',4"-triaminotriphenylmethane (TPM-PS). Examples of the diphenylamine chromogens include N-(carboxymethylaminocarbonyl)-4,4'-bis(dimethylamino)diphenylamine sodium salt (DA-64), 4,4'-bis(dimethylamino)diphenylamine, and bis[3-bis(4-chlorophenyl)methyl-4-dimethylaminophenyl]amine (BCMA).

In the case the detectable substance is light (luminescence), examples of the reagent for measuring hydrogen peroxide include reagents comprising a peroxidatively active substance such as peroxidase and a chemiluminescent substance. Examples of the chemiluminescent substance include luminol, isoluminol, lucigenin, and acridinium ester.

In the case the detectable substance is fluorescence, examples of the reagent for measuring hydrogen peroxide

include a reagent comprising a peroxidatively active substance such as peroxidase and a fluorescent substance. Examples of the fluorescent substance include 4-hydroxyphenylacetic acid, 3-(4-hydroxyphenyl)propionic acid, and coumarin.

(2) Reagent for measuring glycated hemoglobin in hemoglobin-containing sample

The reagent for measuring glycated hemoglobin in a hemoglobin-containing sample according to the present invention comprises a protease, fructosyl peptide oxidase, a halogen oxide, and a surfactant. The measuring reagent of the present invention is used in the method for measuring glycated hemoglobin in a hemoglobin-containing sample according to the present invention. The measuring reagent of the present invention can further comprise a reagent for measuring hydrogen peroxide.

Examples of the protease, the fructosyl peptide oxidase, the halogen oxide, the surfactant, and the reagent for measuring hydrogen peroxide in the measuring reagent of the present invention include the aforementioned protease, fructosyl peptide oxidase, halogen oxide, surfactant, and reagent for measuring hydrogen peroxide, respectively.

A concentration of the protease in the measuring reagent of the present invention is usually 50 to 25,000 kU/L, preferably 250 to 10,000 kU/L. A concentration of the fructosyl peptide oxidase in the measuring reagent of the present invention is usually 0.1 to 30 kU/L, preferably 0.2 to 15 kU/L.

A concentration of the halogen oxide in the measuring reagent of the present invention is usually 0.005 to 20 mmol/L, preferably 0.01 to 10 mmol/L.

A concentration of the surfactant in the measuring reagent of the present invention is usually 0.0001 to 10%, preferably 0.0005 to 5%.

The measuring reagent of the present invention can optionally comprise an aqueous medium, a stabilizer, an antiseptic, salts, an interference inhibitor, an organic solvent, and the like.

Examples of the aqueous medium include a deionized water, a distilled water, and a buffer solution. A buffer solution is preferable.

The pH of the aqueous medium is, for example, 4 to 10. In the case of using a buffer solution as the aqueous medium, a buffer is preferably used according to the set pH. Examples of the buffer used in the buffer solution include a tris(hydroxymethyl)aminomethane buffer, a phosphate buffer, a borate buffer, and a Good's buffer.

Examples of the Good's buffer include 2-morpholinoethanesulfonic acid (MES), bis(2-hydroxyethyl)iminotris(hydroxymethyl)methane (Bis-Tris), N-(2-acetamido)iminodiacetic acid (ADA), piperazine-N,N'-bis(2-ethanesulfonic acid) (PIPES), N-(2-acetamido)-2-aminoethanesulfonic acid (ACES), 3-morpholino-2-hydroxypropanesulfonic acid (MOPSO), N,N-bis(2-hydroxyethyl)-2-aminoethanesulfonic acid (BES), 3-morpholinopropanesulfonic acid (MOPS), N-[tris(hydroxymethyl)methyl]-2-aminoethanesulfonic acid (TES), 2-[4-(2-hydroxyethyl)-1-piperazinyl]ethanesulfonic acid (HEPES), 3-[N,N-bis(2-hydroxyethyl)amino]-2-hydroxypropanesulfonic acid (DIPSO), N-[tris(hydroxymethyl)methyl]-2-hydroxy-3-aminopropanesulfonic acid (TAPSO), piperazine-N,N'-bis(2-hydroxypropanesulfonic acid) (POPSO), 3-[4-(2-hydroxyethyl)-1-piperazinyl]-2-hydroxypropanesulfonic acid (HEPPSO), 3-[4-(2-hydroxyethyl)-1-piperazinyl]propanesulfonic acid [(H)EPPS], N-[tris(hydroxymethyl)methyl]glycine (Tricine), N,N-bis(2-hydroxyethyl)glycine (Bicine), N-tris(hydroxymethyl)methyl-3-aminopropanesulfonic acid (TAPS), N-cyclohexyl-2-aminoethanesulfonic acid (CHES), N-cyclohexyl-3-amino-2-hydroxypropanesulfonic acid (CAPSO), and N-cyclohexyl-3-aminopropanesulfonic acid (CAPS).

A concentration of the buffer solution is usually 0.001 to 2.0 mol/L, preferably 0.005 to 1.0 mol/L.

Examples of the stabilizer include ethylenediaminetetraacetic acid (EDTA), sucrose, calcium chloride, calcium acetate, potassium ferrocyanide, bovine serum albumin (BSA), and a polyoxyethylene surfactant. Examples of the polyoxyethylene surfactant include polyoxyethylene alkylphenyl ether [commercially available products: Nonion HS-240(manufactured by NOF Corp.) and Triton X-405 (manufactured by Sigma-Aldrich Corp.)]. Examples of the antiseptic include sodium azide and an antibiotic. Examples of the salt include sodium chloride, sodium nitrate, sodium sulfate, sodium carbonate, potassium chloride, potassium nitrate, potassium sulfate, and potassium carbonate. Examples of the interference inhibitor include an ascorbic acid oxidase for eliminating the influence of ascorbic acid. Examples of the organic solvent include dimethylformamide (DMF), dimethyl sulfoxide (DMSO), dioxane, acetone, methanol, and ethanol that make the leuco chromogen soluble in the aqueous medium.

(3) Kit for measuring glycated hemoglobin in hemoglobin-containing sample

The reagent for measuring glycated hemoglobin in a hemoglobin-containing sample according to the present invention can be stored, distributed, and used in the form of a kit. The kit for measuring glycated hemoglobin in a hemoglobin-containing sample according to the present invention is used in the method for measuring glycated hemoglobin in a hemoglobin-containing sample according to the present invention. Examples of the measuring kit of the present invention include a kit consisting of two reagents and a kit consisting of three reagents. A kit consisting of two reagents is preferable.

The kit for measuring glycated hemoglobin in a hemoglobin-containing sample according to the present invention is not particularly limited as long as the kit enables the method for measuring glycated hemoglobin in a hemoglobin-containing sample according to the present invention. Examples of the kit consisting of two reagents include a kit comprising: a first reagent comprising a protease; and a second reagent comprising a fructosyl peptide oxidase, a halogen oxide, and a surfactant; and a kit comprising: a first reagent comprising a protease, a halogen oxide, and a surfactant; and a second reagent comprising a fructosyl peptide oxidase.

Another example of the kit for measuring glycated hemoglobin according to the present invention include the kits described above, wherein either the first reagent or the second reagent, or both of the first reagent and the second reagent, comprise a reagent for measuring hydrogen peroxide. In particular, in the case of using a reagent for measuring hydrogen peroxide comprising peroxidase and a leuco chromogen, the peroxidase and the leuco chromogen are preferably contained in separate reagents. Specifically, the peroxidase and the leuco chromogen are preferably contained in the first reagent and the second reagent or the second reagent and the first reagent, respectively.

A concentration of the protease in the reagent constituting the measuring kit of the present invention is usually 100 to 30000 kU/L, preferably 500 to 10000 kU/L. A concentration of the fructosyl peptide oxidase in the reagent constituting the measuring kit of the present invention is usually 0.5 to 100 kU/L, preferably 1 to 50 kU/L.

A concentration of the halogen oxide in the reagent constituting the measuring kit of the present invention is usually 0.005 to 20 mmol/L, preferably 0.01 to 10 mmol/L.

A concentration of the surfactant in the reagent constituting the measuring kit of the present invention is usually 0.0001 to 40%, preferably 0.0005 to 20%.

Hereinafter, the present invention will be described in more detail with reference to Examples. However, the scope of the present invention is not limited to these examples by any means.

In Examples, Comparative Examples, and Test Examples below, reagents and enzymes from the following manufacturers were used.

ACES (manufactured by Dojindo Laboratories), ADA (manufactured by Dojindo Laboratories), calcium acetate monohydrate (manufactured by Wako Pure Chemical Industries, Ltd.), sodium chloride (manufactured by Wako Pure Chemical Industries, Ltd.), DA-67 (manufactured by Wako Pure Chemical Industries, Ltd.), 1-dodecylpyridinium chloride (C12py) (compound (I); manufactured by Tokyo Chemical Industry Co., Ltd.), 1-cetylpyridinium chloride (C16py) (compound (I); manufactured by Tokyo Chemical Industry Co., Ltd.), tributyldodecylphosphonium bromide (C12TBP) (compound (II); manufactured by Tokyo Chemical Industry Co., Ltd.), tetradecyltrimethylammonium chloride (C14TMA) [compound (III); manufactured by Tokyo Chemical Industry Co., Ltd.], potassium iodate (halogen oxide; manufactured by Tokyo Chemical Industry Co., Ltd.), potassium bromate (halogen oxide; manufactured by Tokyo Chemical Industry Co., Ltd.), potassium periodate (halogen oxide; manufactured by Tokyo Chemical Industry Co., Ltd.), Nonion HS-240 (polyoxyethylene alkylphenyl ether; manufactured by NOF Corp.), Thermolysin (protease; manufactured by Daiwa Fine Chemicals Co., Ltd.), FPOX-CE (fructosyl peptide oxidase; manufactured by Kikkoman Corp.), FPOX-CET (fructosyl peptide oxidase; manufactured by Kikkoman Corp.), and peroxidase (manufactured by Toyobo Co., Ltd.).

Example 1

A kit for measuring HbA1c consisting of the following first and second reagents was prepared.

First reagent

| | |
|---|---|
| ACES (pH 7.0) | 20 mmol/L |
| Calcium acetate monohydrate | 10 mmol/L |
| Sodium chloride | 100 mmol/L |
| C12py | 1.6 g/L |
| Potassium iodate | 0.1 g/L |
| Thermolysin | 1,800 kU/L |
| DA-67 | 25 μmol/L |

Second reagent

| | |
|---|---|
| ADA (pH 7.0) | 50 mmol/L |
| Nonion HS-240 | 5 g/L |
| FPOX-CE | 12 kU/L |
| Peroxidase | 120 kU/L |

Example 2

A kit for measuring HbA1c consisting of the following first and second reagents was prepared.

First reagent

| | |
|---|---|
| ACES (pH 7.0) | 20 mmol/L |
| Calcium acetate monohydrate | 10 mmol/L |
| Sodium chloride | 100 mmol/L |
| C12py | 1.6 g/L |
| Potassium bromate | 0.1 g/L |
| Thermolysin | 1,800 kU/L |
| DA-67 | 25 μmol/L |

Second reagent

| | |
|---|---|
| ADA (pH 7.0) | 50 mmol/L |
| Nonion HS-240 | 5 g/L |
| FPOX-CE | 12 kU/L |
| Peroxidase | 120 kU/L |

Example 3

A kit for measuring HbA1c consisting of the following first and second reagents was prepared.
First reagent

| | |
|---|---|
| ACES (pH 7.0) | 20 mmol/L |
| Calcium acetate monohydrate | 10 mmol/L |
| Sodium chloride | 100 mmol/L |
| C12py | 1.6 g/L |
| Potassium periodate | 0.1 g/L |
| Thermolysin | 1,800 kU/L |
| DA-67 | 25 μmol/L |

Second reagent

| | |
|---|---|
| ADA (pH 7.0) | 50 mmol/L |
| Nonion HS-240 | 5 g/L |
| FPOX-CE | 12 kU/L |
| Peroxidase | 120 kU/L |

Example 4

A kit for measuring HbA1c consisting of the following first and second reagents was prepared.

First reagent

| | |
|---|---|
| ACES (pH 7.0) | 20 mmol/L |
| Calcium acetate monohydrate | 10 mmol/L |
| Sodium chloride | 100 mmol/L |
| C16py | 0.35 g/L |
| Potassium bromate | 0.025 g/L |
| Thermolysin | 1,800 kU/L |
| DA-67 | 25 μmol/L |

Second reagent

| | |
|---|---|
| ADA (pH 7.0) | 50 mmol/L |
| Nonion HS-240 | 5 g/L |
| FPOX-CET | 6 kU/L |
| Peroxidase | 120 kU/L |

Example 5

A kit for measuring HbA1c consisting of the following first and second reagents was prepared. First reagent

| | |
|---|---|
| ACES (pH 7.0) | 20 mmol/L |
| Calcium acetate monohydrate | 10 mmol/L |
| Sodium chloride | 100 mmol/L |
| C12TBP | 0.8 g/L |
| Potassium bromate | 0.1 g/L |
| Thermolysin | 1,800 kU/L |
| DA-67 | 25 μmol/L |

Second reagent

| | |
|---|---|
| ADA (pH 7.0) | 50 mmol/L |
| Nonion HS-240 | 5 g/L |
| FPOX-CET | 6 kU/L |
| Peroxidase | 120 kU/L |

Example 6

A kit for measuring HbA1c consisting of the following first and second reagents was prepared.

First reagent

| | |
|---|---|
| ACES (pH 7.0) | 20 mmol/L |
| Calcium acetate monohydrate | 10 mmol/L |
| Sodium chloride | 100 mmol/L |
| C14TMA | 0.6 g/L |
| Potassium iodate | 0.1 g/L |
| Thermolysin | 1,800 kU/L |
| DA-67 | 25 μmol/L |

Second reagent

| | |
|---|---|
| ADA (pH 7.0) | 50 mmol/L |
| Nonion HS-240 | 5 g/L |
| FPOX-CE | 12 kU/L |
| Peroxidase | 120 kU/L |

Example 7

A kit for measuring HbA1c consisting of the following first and second reagents was prepared.

First reagent

| | |
|---|---|
| ACES (pH 7.0) | 20 mmol/L |
| Calcium acetate monohydrate | 10 mmol/L |
| Sodium chloride | 100 mmol/L |
| C14TMA | 0.6 g/L |
| Potassium periodate | 0.1 g/L |
| Thermolysin | 1,800 kU/L |
| DA-67 | 25 μmol/L |

Second reagent

| | |
|---|---|
| ADA (pH 7.0) | 50 mmol/L |
| Nonion HS-240 | 5 g/L |
| FPOX-CE | 12 kU/L |
| Peroxidase | 120 kU/L |

Example 8

The kit of Example 1 was used as a kit for measuring HbA1c. Whole blood derived from 10 test subjects suspected of having diabetes mellitus was used as a sample to determine the ratio [HbA1c (%)] of HbA1c concentration (amount) to total hemoglobin concentration (amount) in each of the samples by the following procedures:

(1) Preparation of calibration curve for determining total hemoglobin concentration

"Hemoglobin B-Test Wako" (SLS-hemoglobin method) (manufactured by Wako Pure Chemical Industries, Ltd.) was used as a kit for measuring total hemoglobin. A standard (hemoglobin concentration: 15.3 mg/mL) included in "Hemoglobin B-Test Wako" was used as a specimen in measurement to prepare a calibration curve showing the relationship between hemoglobin concentration and absorbance.

(2) Preparation of calibration curve for determining HbA1c concentration

For each of two blood cell fractions with HbA1c concentrations valued at 2.77 μmol/L and 6.33 μmol/L, respectively, by latex immunoagglutination assay and the total hemoglobin value for each of the blood cell fractions, the measurement was performed using the kit for measuring HbA1c of Example 1 to determine the absorbance for each of the blood cell fractions. Saline was used instead of the blood cell fraction to determine absorbance. The absorbance for the saline was subtracted from the absorbance for each of the blood cell fractions, and the value thus calculated was used as the blank-corrected absorbance for the blood cell fraction. A calibration curve showing the relationship between HbA1c concentration (μmol/L) and absorbance was prepared based on the blank-corrected absorbance for the blood cell fraction and the blank-corrected absorbance (0 Abs) for the saline.

(3) Determination of hemoglobin concentration in each of blood cell fractions

Each of the samples was centrifuged at 3,000 rpm at 25°C for 5 minutes to obtain a blood cell fraction. For each of the blood cell fractions, the measurement was performed using "Hemoglobin B-Test Wako" to determine absorbance. The hemoglobin concentration (μmol/L) in each of the blood cell fractions was determined based on the obtained measurement value and the calibration curve prepared in (1).

(4) Determination of HbA1c concentration in each of blood cell fractions

For each of the blood cell fractions, the measurement was performed using the measuring kit of Example 1 to determine absorbance. The HbA1c concentration (μmol/L) in each of the blood cell fractions was determined based on the obtained measurement value and the calibration curve prepared in (2).

(5) Determination of HbA1c (%) (= ratio of HbA1c concentration to hemoglobin concentration)

HbA1c (%) was calculated as a Japan Diabetes Society (JDS) value according to the following formula based on the hemoglobin concentration (μmol/L) in each of the blood cell fractions determined in (3) and the HbA1c concentration (μmol/L) in each of the blood cell fractions determined in (4):

[Equation 1]

$$\mathrm{HbA1c}(\%) = [\mathrm{HbA1c\ concentration}\ (\mu\mathrm{mol/L})]/[\mathrm{Hemoglobin\ concentration}\ (\mu\mathrm{mol/L})] \times 0.0963 + 1.62$$

(6) Determination of HbA1c (%) in the same blood cell fraction by immunoassay

The same blood cell fractions as those used in the determination of HbA1c (%) in (5) were used. HbA1c (%) in each of the blood cell fractions was determined by immunoassay using "Determiner L HbA1c" (manufactured by Kyowa Medex Co., Ltd.) according to the protocol described in the attachment of "Determiner L HbAlc".

(7) Correlation between measuring method of the present invention and immunoassay

The correlation between the measuring method of the present invention and immunoassay was verified from HbA1c (%) determined in (5) using the measuring method of the present invention and HbA1c (%) determined in (6) using the immunoassay to determine a correlation coefficient.

The correlation coefficient between the measuring method of the present invention and measurement using "Determiner L HbA1c" (manufactured by Kyowa Medex Co., Ltd.) was determined by the same procedures as above using the measuring kits of Examples 2 to 7 instead of the measuring kit of Example 1. The results are shown in Table 1.

[Table 1]

Table 1

| Kit | Correlation coefficient |
|---|---|
| Example 1 | **0.979** |
| Example 2 | **0.983** |
| Example 3 | **0.977** |
| Example 4 | **0.999** |
| Example 5 | **0.996** |
| Example 6 | **0.996** |
| Example 7 | **0.996** |

As shown in Table 1, favorable correlation was confirmed between the measuring method of the present invention using each of the measuring kits of Examples 1 to 7 and the immunoassay. These results demonstrated that the measuring method of the present invention using each of the measuring kits of Examples 1 to 7 could accurately and highly sensitively measure HbA1c in a sample.

[Comparative Example 1]

A kit for measuring HbA1c consisting of the following first and second reagents was prepared.

First reagent

| | |
|---|---|
| ACES (pH 7.0) | 20 mmol/L |
| Calcium acetate monohydrate | 10 mmol/L |
| Sodium chloride | 100 mmol/L |
| C12py | 1.6 g/L |
| Thermolysin | 1,800 kU/L |
| DA-67 | 25 μmol/L |

Second reagent

| | |
|---|---|
| ADA (pH 7.0) | 50 mmol/L |
| Nonion HS-240 | 5 g/L |
| FPOX-CE | 12 kU/L |
| Peroxidase | 120 kU/L |

[Comparative Example 2]

A kit for measuring HbA1c consisting of the following first and second reagents was prepared.

First reagent

| | |
|---|---|
| ACES (pH 7.0) | 20 mmol/L |
| Calcium acetate monohydrate | 10 mmol/L |
| Sodium chloride | 100 mmol/L |
| C16py | 0.35 g/L |

(continued)

| | |
|---|---|
| Thermolysin | 1,800 kU/L |
| DA-67 | 25 μmol/L |

Second reagent

| | |
|---|---|
| ADA (pH 7.0) | 50 mmol/L |
| Nonion HS-240 | 5 g/L |
| FPOX-CET | 6 kU/L |
| Peroxidase | 120 kU/L |

[Comparative Example 3]

A kit for measuring HbA1c consisting of the following first and second reagents was prepared. First reagent

| | |
|---|---|
| ACES (pH 7.0) | 20 mmol/L |
| Calcium acetate monohydrate | 10 mmol/L |
| Sodium chloride | 100 mmol/L |
| C12TBP | 0.8 g/L |
| Thermolysin | 1,800 kU/L |
| DA-67 | 25 μmol/L |

Second reagent

| | |
|---|---|
| ADA (pH 7.0) | 50 mmol/L |
| Nonion HS-240 | 5 g/L |
| FPOX-CET | 6 kU/L |
| Peroxidase | 120 kU/L |

[Comparative Example 4]

A kit for measuring HbA1c consisting of the following first and second reagents was prepared.

First reagent

| | |
|---|---|
| ACES (pH 7.0) | 20 mmol/L |
| Calcium acetate monohydrate | 10 mmol/L |
| Sodium chloride | 100 mmol/L |
| C14TMA | 0.6 g/L |
| Thermolysin | 1,800 kU/L |
| DA-67 | 25 μmol/L |

Second reagent

| | |
|---|---|
| ADA (pH 7.0) | 50 mmol/L |

(continued)

| | |
|---|---|
| Nonion HS-240 | 5 g/L |
| FPOX-CE | 12 kU/L |
| Peroxidase | 120 kU/L |

[Test Example 1] Effect of halogen oxide - Suppression of influence of hemoglobin concentration

(1) Preparation of hemolyzed specimen

For a blood cell fraction obtained by the centrifugation of human blood, the absorbance was measured using a hemoglobin measuring reagent "Nescoat Hemo Kit-N" (manufactured by Alfresa Pharma Corp.) to determine the concentration of hemoglobin in the blood cell fraction. Subsequently, the blood cell fraction thus valued was hemolyzed by dilution with purified water to prepare each of the hemolyzed specimens having a hemoglobin concentration of 2 mg/mL, 4 mg/mL, 6 mg/mL, 8 mg/mL, or 10 mg/mL.

(2) Determination of HbA1c (%) for hemolyzed specimen

The kit of Example 1 was used as a kit. Each of the hemolyzed specimens prepared in (1) was used as a specimen in the measurement. On the basis of the obtained measurement value, the HbA1c concentration (μmol/L) of each of the specimens was determined based on the calibration curve showing the relationship between HbA1c concentration (μmol/L) and absorbance, prepared in Example 8(2).

Meanwhile, the absorbance for the same hemolyzed specimen as above was determined using "Hemoglobin B-Test Wako". The hemoglobin concentration (μmol/L) of each of the specimens was determined from the obtained measurement value and the calibration curve prepared in Example 8(1).

HbA1c (%) was calculated as a Japan Diabetes Society (JDS) value according to the following equation from the determined HbA1c concentration (μmol/L) and hemoglobin concentration (μmol/L) of each specimen:

[Equation 2]

$$\text{HbA1c}(\%) = [\text{HbA1c concentration}(\mu\text{mol/L})]/[\text{Hemoglobin concentration}(\mu\text{mol/L})] \times 0.0963 + 1.62$$

HbA1c (%) in each of the specimens was determined by the same measurement using each of the kits except that the kits of Examples 2 to 7 and Comparative Examples 1 to 4 were separately used instead of the kit of Example 1. The HbA1c (%) of the specimen with a hemoglobin concentration of 6 mg/mL was used as a reference 0. A difference [ΔHbA1c (%)] of the HbA1c (%) from the reference was calculated for each of the specimens. The results are shown in Table 2.

[Table 2]

Table 2

| Kit | ΔHbA1c (%) | | | | |
|---|---|---|---|---|---|
| | Specimen [hemoglobin concentration (mg/mL)] | | | | |
| | 2 | 4 | 6 | 8 | 10 |
| Example 1 | 0.9 | 0.2 | 0.0 | 0.0 | -0.1 |
| Example 2 | -0.1 | -0.3 | 0.0 | 0.1 | -0.1 |
| Example 3 | 0.9 | 0.2 | 0.0 | 0.0 | -0.2 |
| Comparative Example 1 | 1.6 | 0.5 | 0.0 | -0.2 | -0.3 |
| Example 4 | 0.3 | 0.3 | 0.0 | -0.2 | -0.3 |
| Comparative Example 2 | 2.4 | 0.4 | 0.0 | -0.2 | -0.4 |
| Example 5 | -0.3 | 0.1 | 0.0 | -0.1 | -0.3 |
| Comparative Example 3 | 1.3 | 0.2 | 0.0 | -0.3 | -0.5 |
| Example 6 | 0.3 | -0.1 | 0.0 | -0.1 | -0.1 |
| Example 7 | 0.2 | 0.1 | 0.0 | 0.0 | 0.0 |
| Comparative Example 4 | 0.9 | 0.3 | 0.0 | 0.0 | -0.2 |

Since the hemolyzed specimens used in the measurement were prepared from the same human blood, the ratio (%) of HbA1c to total hemoglobin is constant, irrespective of hemoglobin concentration. Thus, ΔHbA1c (%) closer to 0 means that the measuring method of HbA1c is less influenced by hemoglobin concentration. As is evident from Table 2, the kit of the present invention comprising the halogen oxide was shown to be insusceptible to hemoglobin concentration compared with the kit of Comparative Example comprising no halogen oxide.

[Test Example 2] Effect of halogen oxide (2) - High sensitivity measurement

Each of the kits of Examples 1 to 3 and Comparative Example 1 was used as a kit. Each of the hemolyzed specimens prepared in Test Example 1(1) was used as a specimen. The reaction absorbance for each of the specimens was determined in the same way as in Test Example 1 using each of the kits. The results are shown in Figure 1.

Similarly, each of the kits of Example 4 and Comparative Example 2 was used as a kit, and each of the hemolyzed specimens prepared in Test Example 1(1) was used as a specimen. The reaction absorbance for each of the specimens was determined in the same way as in Test Example 1 using each of the kits. The results are shown in Figure 2.

Similarly, each of the kits of Example 5 and Comparative Example 3 was used as a kit, and each of the hemolyzed specimens prepared in Test Example 1(1) was used as a specimen. The reaction absorbance for each of the specimens was determined in the same way as in Test Example 1 using each of the kits. The results are shown in Figure 3.

Similarly, each of the kits of Example 6 and Comparative Example 4 was used as a kit, and each of the hemolyzed specimens prepared in Test Example 1(1) was used as a specimen. The reaction absorbance for each of the specimens was determined in the same way as in Test Example 1 using each kit. The results are shown in Figure 4.

As is evident from Figures 1 to 4, the reaction absorbance increased in proportion to hemoglobin concentration in both of the kit of the present invention and the kit of Comparative Example. However, the reaction absorbance was shown to be higher in the specimens having the same concentration of hemoglobin (i.e., the specimens having the same concentration of HbAlc) using the kit of the present invention comprising the halogen oxide than using the kit of Comparative Example comprising no halogen oxide.

Since the hemolyzed specimens used in the measurement were prepared from the same human blood, as described above, the HbA1c concentration increases depending on the hemoglobin concentration. In the specimens having the same concentration of HbA1c, the higher reaction absorbance means that measurement can be carried out with higher sensitivity.

The high reaction absorbance was obtained in the specimens having the same concentration of HbA1c using

each of the kits of Examples 1 to 3 compared with the kit of Comparative Example 1. Similarly, the high reaction absorbance was obtained in the specimens having the same concentration of HbA1c using the kits of Examples 4, 5, and 6 compared with the kits of Comparative Examples 2, 3, 4, respectively. These results demonstrated that the measuring method using the kit of the present invention comprising the halogen oxide enables highly sensitive measurement of HbA1c, compared with the measuring method using the kit of Comparative Example comprising no halogen oxide.

**Industrial Applicability**

The present invention provides a method, a reagent, and a kit for measuring glycated hemoglobin in a hemoglobin-containing sample, which are useful in, for example, measuring glycated hemoglobin useful in the diagnosis of diabetes mellitus.

## Claims

**1.** A method for measuring glycated hemoglobin in a hemoglobin-containing sample comprising: reacting hemoglobin-containing sample with a protease in the presence of a surfactant, then reacting the obtained reaction product with fructosyl peptide oxidase, wherein the latter reaction or both of the former reaction and the latter reaction are performed in the presence of a halogen oxide, and measuring the generated hydrogen peroxide.

**2.** The method according to claim 1, wherein the halogen oxide is a halogen oxide selected from the group consisting of iodic acid or a salt thereof, bromic acid or a salt thereof, and periodic acid or a salt thereof.

**3.** The method according to claim 1 or 2, wherein the surfactant is a cationic surfactant.

**4.** The method according to claim 3, wherein the cationic surfactant is a cationic surfactant selected from the group consisting of a pyridinium salt represented by the following formula (I), a phosphonium salt represented by the following formula (II), and a quaternary ammonium salt represented by the following formula (III):

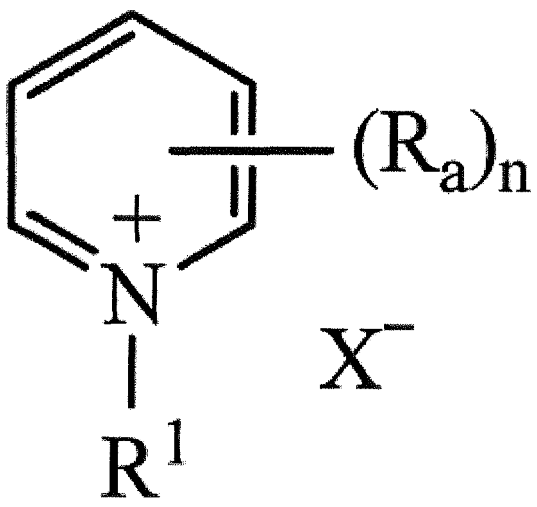

(I)

wherein $R^1$ represents a substituted or unsubstituted alkyl or a substituted or unsubstituted alkenyl; $R_a$ represents a hydrogen atom, a substituted or unsubstituted alkyl, or a substituted or unsubstituted alkenyl; n represents an integer of 1 to 5; and $X^-$ represents a monovalent anion,

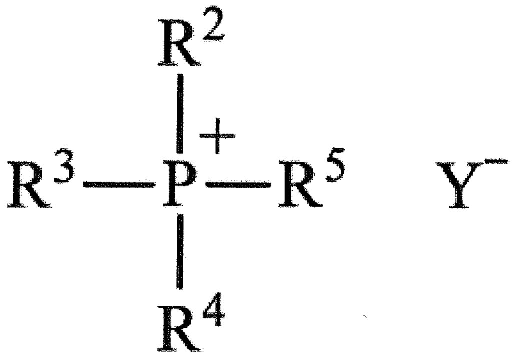

(II)

wherein $R^2$ to $R^5$ are the same or different, and each represents a substituted or unsubstituted alkyl; and $Y^-$ represents a monovalent anion, and

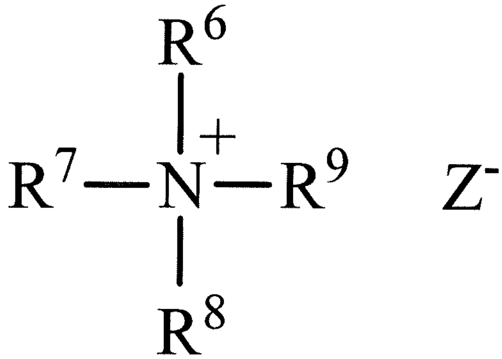

(III)

wherein $R^6$ to $R^9$ are the same or different, and each represents a substituted or unsubstituted alkyl; and $Z^-$ represents a monovalent anion.

**5.** The method according to any one of claims 1 to 4, wherein the measurement of the hydrogen peroxide is performed using a reagent for measuring hydrogen peroxide.

**6.** The method according to claim 5, wherein the reagent for measuring hydrogen peroxide is a reagent comprising peroxidase and a leuco chromogen.

**7.** A reagent for measuring glycated hemoglobin in a hemoglobin-containing sample comprising a protease, fructosyl peptide oxidase, a halogen oxide, and a surfactant.

**8.** The reagent according to claim 7, wherein the halogen oxide is a halogen oxide selected from the group consisting of iodic acid or a salt thereof, bromic acid or a salt thereof, and periodic acid or a salt thereof.

**9.** The reagent according to claim 7 or 8, wherein the surfactant is a cationic surfactant.

**10.** The reagent according to claim 9, wherein the cationic surfactant is a cationic surfactant selected from the group consisting of a pyridinium salt represented by the following formula (I), a phosphonium salt represented by the following formula (II), and a quaternary ammonium salt represented by the following formula (III):

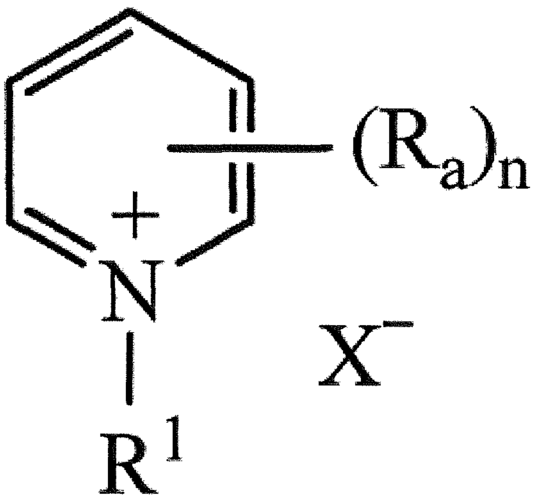

(I)

wherein $R^1$ represents a substituted or unsubstituted alkyl or a substituted or unsubstituted alkenyl; $R_a$ represents a hydrogen atom, a substituted or unsubstituted alkyl, or a substituted or unsubstituted alkenyl; n represents an integer of 1 to 5; and $X^-$ represents a monovalent anion,

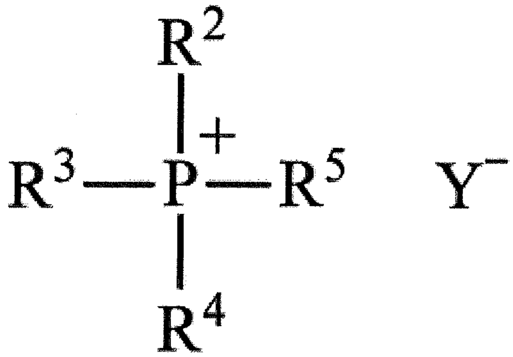

(II)

wherein $R^2$ to $R^5$ are the same or different, and each represents a substituted or unsubstituted alkyl; and $Y^-$represents a monovalent anion, and

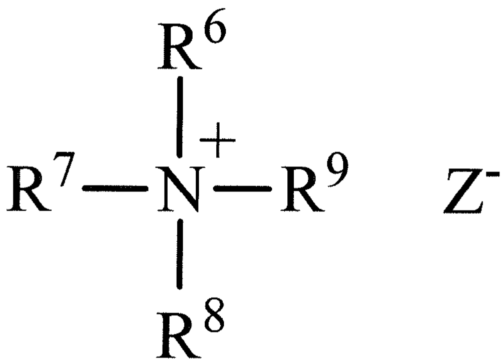

(III)

wherein $R^6$ to $R^9$ are the same or different, and each represents a substituted or unsubstituted alkyl; and $Z^-$ represents a monovalent anion.

**11.** The reagent according to any one of claims 7 to 10, further comprising a reagent for measuring hydrogen peroxide.

**12.** The reagent according to claim 11, wherein the reagent for measuring hydrogen peroxide is a reagent comprising peroxidase and a leuco chromogen.

**13.** A kit for measuring glycated hemoglobin in a hemoglobin-containing sample comprising: a first reagent comprising a protease, a halogen oxide, and a surfactant; and a second reagent comprising fructosyl peptide oxidase.

**14.** A kit for measuring glycated hemoglobin in a hemoglobin-containing sample comprising: a first reagent comprising a protease and a surfactant; and a second reagent comprising fructosyl peptide oxidase and a halogen oxide.

**15.** The kit according to claim 13 or 14, wherein the halogen oxide is a halogen oxide selected from the group consisting of iodic acid or a salt thereof, bromic acid or a salt thereof, and periodic acid or a salt thereof.

**16.** The kit according to any one of claims 13 to 15, wherein the surfactant is a cationic surfactant.

**17.** The kit according to claim 16, wherein the cationic surfactant is a cationic surfactant selected from the group consisting of a pyridinium salt represented by the following formula (I), a phosphonium salt represented by the following formula (II), and a quaternary ammonium salt represented by the following formula (III):

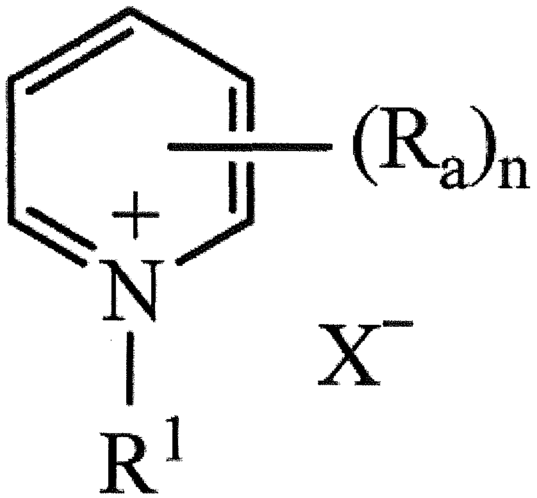

(I)

wherein $R^1$ represents a substituted or unsubstituted alkyl or a substituted or unsubstituted alkenyl; $R_a$ represents a hydrogen atom, a substituted or unsubstituted alkyl, or a substituted or unsubstituted alkenyl; n represents an integer of 1 to 5; and $X^-$ represents a monovalent anion,

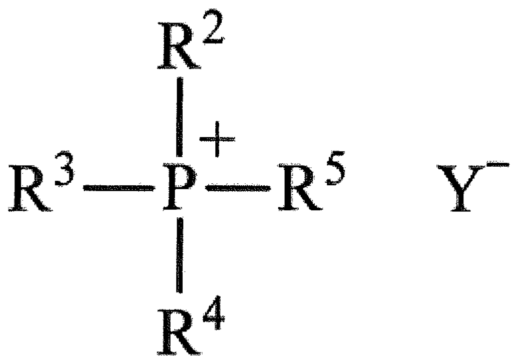

(II)

wherein $R^2$ to $R^5$ are the same or different, and each represents a substituted or unsubstituted alkyl; and $Y^-$ represents a monovalent anion, and

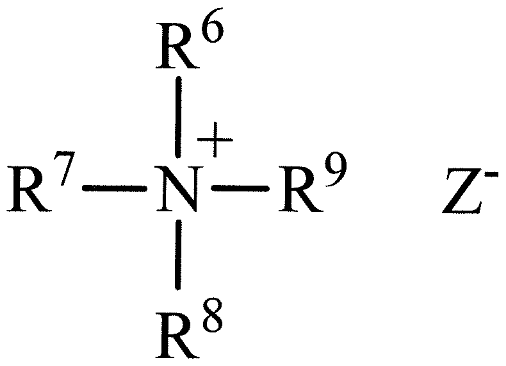

( III )

wherein $R^6$ to $R^9$ are the same or different, and each represents a substituted or unsubstituted alkyl; and $Z^-$ represents a monovalent anion.

**18.** The kit according to any one of claims 13 to 17, wherein each of peroxidase and a leuco chromogen is comprised in the first reagent and the second reagent, or the second reagent and the first reagent, respectively.

[Figure 1]

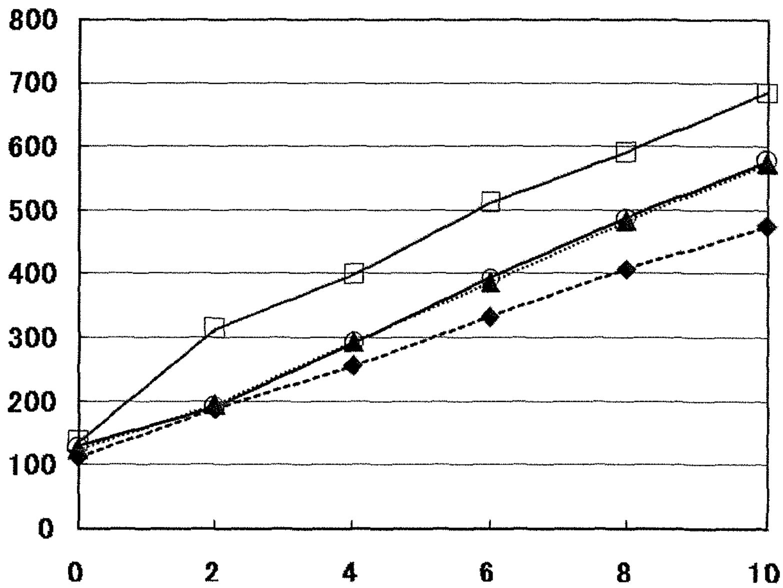

[Figure 2]

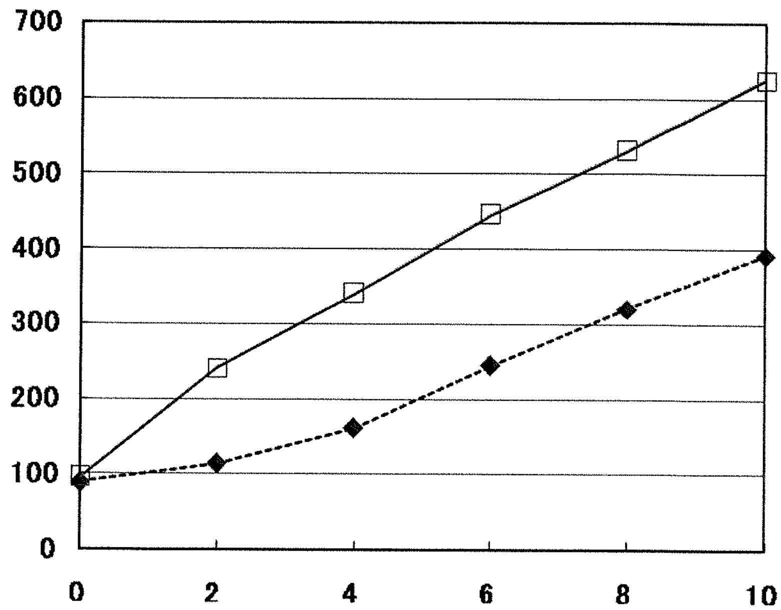

[Figure 3]

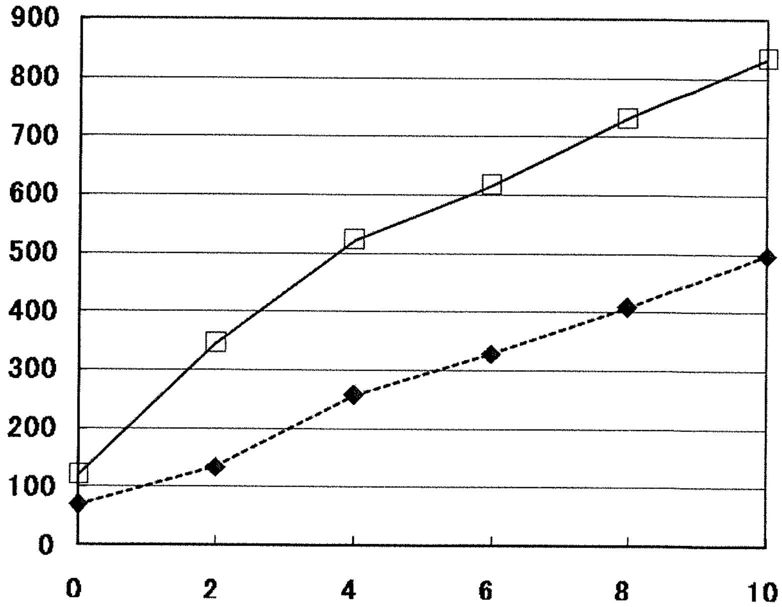

[Figure 4]

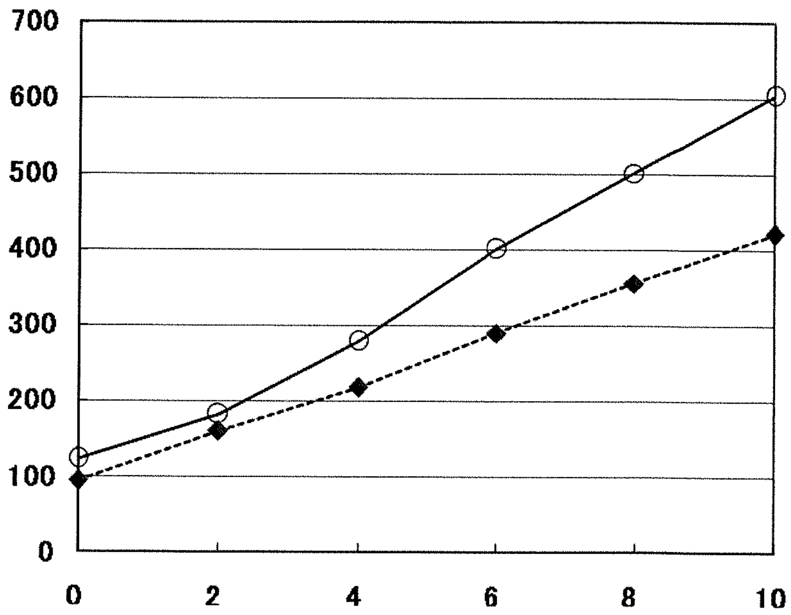

**INTERNATIONAL SEARCH REPORT**

International application No. PCT/JP2012/065237

A. CLASSIFICATION OF SUBJECT MATTER
*C12Q1/37*(2006.01)i, *C12Q1/26*(2006.01)i, *C12Q1/28*(2006.01)i

According to International Patent Classification (IPC) or to both national classification and IPC

B. FIELDS SEARCHED

Minimum documentation searched (classification system followed by classification symbols)
C12Q1/37, C12Q1/26, C12Q1/28

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CAPlus/BIOSIS/MEDLINE/WPIDS(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X | JP 2009-544315 A (General Atomics), 17 December 2009 (17.12.2009), & US 2008/0096230 A1 & WO 2008/013874 A1 | 1-18 |
| X<br>Y | WO 2003/107011 A1 (Arkray, Inc.), 24 December 2003 (24.12.2003), & US 2005/0221415 A1 & EP 1515144 A1 | 1,2,5-8, 11-15,18<br>3,4,9,10,16, 17 |
| Y | JP 2004-141150 A (Sysmex Corp.), 20 May 2004 (20.05.2004), & US 2004/0067575 A1 & EP 1406088 A2 | 3,4,9,10,16, 17 |
| Y | JP 2004-537725 A (Beckman Coulter, Inc.), 16 December 2004 (16.12.2004), & US 2003/0040115 A1 & WO 2003/012426 A1 | 3,4,9,10,16, 17 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>03 August, 2012 (03.08.12) | Date of mailing of the international search report<br>14 August, 2012 (14.08.12) |
| Name and mailing address of the ISA/<br>Japanese Patent Office<br>Facsimile No. | Authorized officer<br><br>Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3010696 A
- WO 2003107011 A
- JP 2007147630 A
- JP 2000210100 A
- WO 2005049858 A
- JP 56151358 A